(19) Europäisches Patentamt
European Patent Office
Office européen des brevets

(11) Veröffentlichungsnummer : **0 073 381**
**B1**

(12) # EUROPÄISCHE PATENTSCHRIFT

(45) Veröffentlichungstag der Patentschrift : **22.10.86**

(51) Int. Cl.⁴ : **C 07 C101/72**, C 07 C101/77, C 07 D209/20, C 07 C 99/12

(21) Anmeldenummer : **82107366.5**

(22) Anmeldetag : **13.08.82**

(54) **Verfahren zur Extraktion von aromatischen Aminosäuren aus wässriger Phase.**

(30) Priorität : **31.08.81 DE 3134901**

(43) Veröffentlichungstag der Anmeldung : **09.03.83 Patentblatt 83/10**

(45) Bekanntmachung des Hinweises auf die Patenterteilung : **22.10.86 Patentblatt 86/43**

(84) Benannte Vertragsstaaten : **AT BE CH DE FR GB IT LI LU NL SE**

(56) Entgegenhaltungen :
**DE-A- 3 017 861**
**US-A- 2 681 927**
**US-A- 3 928 431**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Weber, Alfred, Dr.**
**Schützallee 56**
**D-1000 Berlin 37 (DE)**
Erfinder : **Wilke, Detlef, Dr.**
**Mariannenstrasse 35**
**D-1000 Berlin 45 (DE)**
Erfinder : **Kurzidim, Johannes, Dr.**
**Brahmsstrasse 11d**
**D-1000 Berlin 45 (DE)**
Erfinder : **Kennecke, Mario, Dr.**
**Taubertstrasse 31f**
**D-1000 Berlin 33 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 073 381 B1

**Beschreibung**

Die Erfindung betrifft ein Verfahren zur Extraktion der aromatischen Aminosäuren L-Phenylalanin, L-Tyrosin, L-Tryptophan oder L-5-Hydroxytryptophan aus einer wässrigen Mikroorganismenkultur deren Filtrat oder deren Zentrifugat, welches dadurch gekennzeichnet ist, daß man sie mit einem Tensid der allgemeinen Formel

$$R—U—SO_3X$$

worin

R einen Alkylrest mit 4 bis 18 Kohlenstoffatomen,

U eine Kohlenstoff-Schwefel-Bindung oder ein Sauerstoffatom und

X ein Alkalimetallatom bedeuten, versetzt, auf einen pH-Wert von 2,0 bis 2,5 ansäuert, und mit einem jeweils 4 bis 6 Kohlenstoffatome enthaltenden Alkohol, Keton oder Carbonsäurealkylester extrahiert.

Bei der Hydrolyse von Proteinen und insbesondere auch bei der fermentativen Herstellung von Aminosäuren erhält man meist wässrige Phasen in denen unterschiedliche Aminosäuren gelöst sind. Die Trennung derartiger Mischungen ist bekanntlich oft recht problematisch.

Aus der US-Patentschrift 3 928 431 ist ein Verfahren zur Extraktion von 3-(3,4-Dihydroxyphenyl)-L-alanin aus wässrigen Lösungen bekannt, dieses Verfahren ist aber recht aufwendig, da es zuvor eine Ausfällung von Calziumionen und eine Vorextraktion benötigt.

Mit Hilfe des erfindungsgemäßen Verfahrens ist es in einfacher Weise möglich, die obengenannten aromatischen Aminosäuren aus derartigen wässrigen Phasen zu extrahieren, während die übrigen Aminosäuren unter diesen Bedingungen nur im geringen Maße extrahiert werden.

Geeignete wässrige Phasen sind beispielsweise solche, die bei der fermentativen Herstellung von L-Phenylalanin (siehe zum Beispiel Chem. Abstr. 76, P 57 722k, 78, P 122 655y, 78, R 146 210a, 82, P 15 274j und 85, P 121 806f), L-Tyrosin (siehe zum Beispiel Chem. Abstr. 76, P 84 566n, 77, P 60 057y, 78, R 146 210a, 82, P 17 119m und 85, P 121 806f) 3-Hydroxy-L-tyrosin = DOPA (siehe zum Beispiel Chem. Abstr. 76, P 152 037z, 78, P 157 879b, 80, 144 375a, 81 P 3 699f und 83, P 7 116q), L-Tryptophan (siehe zum Beispiel Chem. Abstr. 78, P 56 436z, 78, R 146 220d, 81, P 13 463m, 84, 15 655a) und 5-Hydroxy-L-tryptophan (siehe zum Beispiel Chem. Abstr. 76, P 139 064m, 79, P 103 669, 80, P 13 649v, 80, P 106 876g und 83, 106 645s) erhalten werden. Es ist grundsätzlich möglich die so erhaltenen wässrigen Mikroorganismenkulturen selbst zu extrahieren, meist ist es aber vorteilhafter, diese vor der Extraktion zu filtrieren oder zu zentrifugieren und die erhaltenen Lösungen weiter zu verarbeiten.

Als Tenside der allgemeinen Formel, die sich zur Durchführung des erfindungsgemäßen Verfahrens eignen, seien beispielsweise genannt: das Natrium-dodecylbezolsulfonat, das Natrium-pentansulfonat, das Natrium-hexansulfonat, das Natrium-heptansulfonat, das Natrium-octansulfonat, das Natrium-decansulfonat, das Natrium-dodecansulfonat, das Natrium-dodecylsulfat, das Natrium-heptadecylsulfat und käufliche Tenside, die aus Gemischen derartiger Stoffe bestehen. Ein besonders bevorzugtes Tensid ist das Natrium-dodecylsulfat.

Zur Durchführung des erfindungsgemäßen Verfahrens verwendet man vorzugsweise pro Mol zu extrahierender Aminosäure 1,5 bis 5 und insbesondere 2 bis 4 Mol Tensid der allgemeinen Formel.

Als Lösungsmittel, die sich zur Durchführung des erfindungsgemäßen Verfahrens eignen, seien beispielsweise genannt: der Butylalkohol, der Amylalkohol, der sec.-Amylalkohol, der Isoamylalkohol, der Hexylalkohol, das Methyl-butylketon, das Ethyl-propylketon, das Methyl-isobutylketon, das Ethylacetat, das Ethylpropionat, das Ethylbutyrat, das Propylacetat, und das Butylacetat. Besonders bevorzugt sind das Ethylacetat und das Methyl-isobutylketon.

Die Menge des benötigten Extraktionsmittels ist von der Wahl desselben, von der zu extrahierenden Aminosäure und vom verwendeten Tensid abhängig und muß im Einzelfall in üblicher Weise ermittelt werden.

Die Extraktion wird üblicherweise bei etwa 15 °C bis 40 °C und vorzugsweise bei Raumtemperatur durchgeführt.

Aus dem Extrakt kann die aromatische Aminosäure in an sich bekannter Weise isoliert werden, beispielsweise indem man diesen mit Wasser reextrahiert, die Aminosäure an einen stark sauren Ionenaustauscher bindet und mit alkoholischer Ammoniak-Lösung wieder eluiert. Nach Einengen des Eluates erhält man die Aminosäure, während das Tensid sich in der extrahierten wässrigen Phase befindet und hieraus wiedergewonnen werden kann.

Eine andere Möglichkeit besteht zum Beispiel darin, daß man das Tensid an einen schwach basischen Ionenaustauscher bindet und aus der wässrigen Phase die Aminosäure isoliert. Selbstverständlich ist es auch möglich, den Extrakt oder die bei der Reextraktion erhaltene wässrige Phase einzuengen und die Aminosäure durch Kristallisation zu isolieren.

Die nachfolgenden Beispiele dienen zur näheren Erläuterung des erfindungsgemäßen Verfahrens. Um bei diesen Ausführungsbeispielen vergleichbare Resultate zu erzielen, wurde keine wässrige Mikroorganismenkultur verwendet, sondern ein Nährmedium, welches zur Anzucht von Mikroorganismen häufig verwendet wird.

Beispiel 1

100 ml eines Nährmediums, enthaltend 5 % Glucose, 4 % Cornsteep-Liquor, 2 % Harnstoff 0,03 % Dikaliumhydrogenphosphat 0,02 % Kaliumdihydrogenphosphat und 0,01 % Magnesiumchlorid (alle Angaben beziehen sich auf Gramm pro Liter) werden mit 1,00 g Aminosäure und soviel Natrium-dodecylsulfat versetzt, daß pro Mol Aminosäure 3 Mol Tensid vorliegen. Man säuert die Lösung auf einen pH-Wert von 2,2 an und extrahiert einmal mit 200 ml Ethylacetat. In der organischen Phase wird der Gehalt an Aminosäure mittels halbquantitativer Dünnschichtchromatie ermittelt. Die in diesem Versuch erhaltenen Ergebnisse zeigt die nachfolgende Tabelle.

| Eingesetzte Aminosäure | Extraktionsausbeute |
|---|---|
| Tryptophan | über 90% |
| 5-Hydroxytryptophan | über 90% |
| Phenylalanin | über 90% |
| Tyrosin | ca. 90% |
| Histidin | 10 - 20% |
| Serin | 10 - 20% |
| Lysin | 10 - 20% |
| Prolin | 5 - 10% |
| Alanin | ca. 5% |
| Glutaminsäure | unter 5% |

Beispiel 2

Zu 500 ml des im Beispiel 1 genannten Nährmediums gibt man 2,5 g Tryptophan und 10,0 g Natrium-dodecylsulfat, säuert mit Schwefelsäure auf eine pH-Wert von 2,2 an und extrahiert einmal mit 1 000 ml Ethylacetat. Die organische Phase wird mit 500 ml destilliertem Wasser reextrahiert, der wässrige Extrakt auf pH 2 angesäuert und über eine Säule eines stark sauren Kationenaustauschers (Dowex 50) filtriert. Man wäscht den Ionenaustauscher mit Wasser und dann mit 250 ml 50 %iger 1 normaler alkoholischer Ammoniak-Lösung. Letztere wird im Vakuum zur Trockne eingedampft und ergibt einen Rückstand, der analytisch 91,5 % des eingesetzten Tryptophans enthält.

**Patentansprüche**

1. Verfahren zur Extraktion der aromatischen Aminosäuren L-Phenylalanin, L-Tyrosin, L-Tryptophan oder L-5-Hydroxytryptophan aus einer wässrigen Mikroorganismenkultur deren Filtrat oder deren Zentrifugat, dadurch gekennzeichnet, daß man sie mit einem Tensid der allgemeinen Formel

$$R—U—SO_3X$$

worin
R einen Alkylrest mit 4 bis 18 Kohlenstoffatomen,
U eine Kohlenstoff-Schwefel-Bindung oder ein Sauerstoffatom und
X ein Alkalimetallatom bedeuten, versetzt, auf einen pH-Wert von 2,0 bis 2,5 ansäuert, und mit einem jeweils 4 bis 6 Kohlenstoffatome enthaltenden Alkohol, Keton oder Carbonsäurealkylester extrahiert.

2. Verfahren zur Extraktion der aromatischen Aminosäuren gemäß Patentanspruch 1, dadurch gekennzeichnet, daß man als Tensid ein Natriumalkylsulfat mit 10 bis 18 Kohlenstoffatomen im Alkylrest verwendet.

3. Verfahren zur Extraktion der aromatischen Aminosäuren gemäß Patentanspruch 1, dadurch gekennzeichnet, daß man als Tensid ein Natriumalkylsulfonat mit 6 bis 12 Kohlenstoffatomen im Alkylrest verwendet.

4. Verfahren zur Extraktion der aromatischen Aminosäuren gemäß Patentanspruch 1 bis 3, dadurch gekennzeichnet, daß man pro Mol zu extrahierender Aminosäure 1,5 bis 5 Mol Tensid verwendet.

5. Verfahren zur Extraktion der aromatischen Aminosäuren aus wässriger Phase gemäß Patentanspruch 1 bis 4, dadurch gekennzeichnet, daß man als Extraktionsmittel Ethylacetat oder Methylisobutylketon verwendet.

**Claims**

1. Process for the extraction of the aromatic aminoacids L-phenylaniline, L-tyrosine, L-tryptophan or L-5-hydroxytryptophan from an aqueous micro-organism culture filtrate or centrifugate, characterised in that it is treated with a surfactant of the general formula :

$$R—U—SO_3X$$

wherein

R is an alkyl group of 4 to 18 carbon atoms,

U is a carbon-sulphur bond or an oxygen atom and

X is an alkali-metal atom, is acidified to a pH of 2 to 2.5, and is extracted with an alcohol, ketone or carboxylic acid alkyl ester in each case of 4 to 6 carbon atoms.

2. Process for the extraction of aromatic aminoacids according to claim 1, characterised in that the surfactant employed is a sodium alkylsulphate of 10 to 18 carbon atoms in the alkyl group.

3. Process for the extraction of aromatic aminoacids according to claim 1, characterised in that the surfactant employed is a sodium alkylsulphate of 6 to 12 carbon atoms in the alkyl group.

4. Process for the extraction of aromatic aminoacids according to claims 1 to 3, characterised in that 1.5 to 5 moles of surfactant are employed per mole of extracted aminoacid.

5. Process for the extraction of aromatic aminoacids from aqueous phase according to claims 1 to 4, characterised in that ethyl acetate or methylisobutylketone is employed as extraction medium.

**Revendications**

1. Procédé pour extraire certains amino-acides aromatiques, en l'espèce la L-phénylalanine, la L-tyrosine, le L-tryptophane ou le L-hydroxy-5 tryptophane, à partir d'une culture aqueuse de micro-organismes, ou du filtrat ou du produit de centrifugation d'une telle culture, procédé caractérisé en ce qu'on ajoute à cette culture, à son filtrat ou à son produit de centrifugation un surfactif répondant à la formule générale :

$$R—U—SO_3X$$

dans laquelle

R représente un radical alkyle contenant de 4 à 18 atomes de carbone,

U représente une liaison carbone-soufre ou un atome d'oxygène et

X représente un atome de métal alcalin,

on acidifie à un pH de 2,0 à 2,5, et on extrait avec un alcool, une cétone ou un ester alkylique d'acide carboxylique qui contiennent chacun de 4 à 6 atomes de carbone.

2. Procédé pour extraire les amino-acides aromatiques selon la revendication 1, procédé caractérisé en ce qu'on utilise, comme surfactif, un sulfate d'alkyle et de sodium dont le radical alkyle contient de 10 à 18 atomes de carbone.

3. Procédé pour extraire les amino-acides aromatiques selon la revendication 1, procédé caractérisé en ce qu'on utilise, comme surfactif, un alcane-sulfonate de sodium contenant de 6 à 12 atomes de carbone.

4. Procédé pour extraire les amino-acides aromatiques selon l'une quelconque des revendications 1 à 3, procédé caractérisé en ce qu'on utilise de 1,5 à 5 mol du surfactif par mole de l'amino-acide à extraire.

5. Procédé pour extraire les amino-acides aromatiques à partir d'une phase aqueuse selon l'une quelconque des revendications 1 à 4, procédé caractérisé en ce qu'on utilise, comme agent d'extraction, l'acétate d'éthyle ou la méthyl-isobutyl-cétone.